# EUROPEAN PATENT APPLICATION

(11) **EP 1 439 391 A2**
(43) Date of publication of application: **21.07.2004**
(21) Application number: 04250162.7
(22) Date of filing: 14.01.2004
(51) Int. Cl.: G01N 33/553, C12Q 1/68

(54) **Biosensor systems and methods for determining the presence of biomolecules**

(30) Priority: 15.01.2003 US 342562
(71) Applicant: Agilent Technologies, Inc., Palo Alto, CA 94306 (US)
(72) Inventor: Ihoaka, Seiji, Campbell, CA 95008 (US); Roitman, Daniel B., Mento Park, CA 94025 (US); Killeen, Kevin Patrick, Palo Alto, CA 94303 (US); Moon, Ronald Leslie, Atherton, CA 94027 (US)
(74) Representative: Tollett, Ian

(57) **Abstract**

Methods and systems for determining the presence of biomolecules are disclosed. Briefly described, a representative system provides for a biosensor system 100. The biosensor system 100 includes a biomolecule 115 disposed on a conductive substrate 105 having a nanoparticle 125 disposed thereon. The biosensor system 100 also includes a polymer 155 disposed on the nanoparticle 125. The polymer 155 is catalytically generated on the nanoparticle 125 from a plurality of monomers 135. In addition, the biosensor system 100 includes a detector 140 capable of determining the presence of the biomolecule 115.

## Description

The present invention relates to a method and a biosensor system for determining the presence of biomolecules.

In general, a biosensor is capable of identifying biomolecules such as polynucleotides and polypeptides. One use of a biosensor has been to deposit probes onto a substrate and place a biomolecule sample containing target biomolecules onto the substrate. The probe can be various biomolecules that can interact with target biomolecules, while the substrate can be a solid surface such as silica, surface-derivatized glass, polypropylene, and activated polyacrylamide. Then, the target biomolecules can interact (e.g., bond or hybridize) with one or more of the probes. Subsequently, the interaction can be analyzed based on the presence and location of the target biomolecule-probe interaction, which can be determined by one or more detection techniques such as optical, radiochemical, and electrochemical techniques.

Another way in which a biosensor is used includes depositing target biomolecules onto a substrate and adding a solution. The solution can include complimentary polynucleotide or polypeptide probe samples derived from biomolecules that have been tagged with fluorescent dyes. The probe material interacts selectively with target biomolecues only where complimentary bonding sites occur. In other words, probe biomolecules with similar chemical characteristics (e.g., nucleotide sequence or amino acid sequence) to the target biomolecule interact with the target molecules, while dissimilar probe biomolecules do not significantly interact with the target biomolecules. Thereafter, the presence and quantity of bound probe biomolecules can be detected and analyzed in a manner similar to the detection techniques discussed above.

As stated above, biomolecules can be detected using a variety of detection techniques. However, many of these techniques have disadvantages such as low detection limits, variation in reproducibility of results, and problems with specificity of intercalations. Thus, there is a need in the industry for a biomolecule detection technique that overcomes at least these as well as other disadvantages.

Methods and systems for determining the presence of biomolecules are provided. Briefly described, one exemplary system, among others, includes a first biomolecule disposed on a first conductive substrate having a first nanoparticle disposed thereon. The system also includes a polymer disposed on the first nanoparticle. The polymer is catalytically generated on the first nanoparticle from a plurality of monomers. In addition, the system includes a detector capable of determining the presence of the first biomolecule.

Briefly described is a system that includes a first biomolecule disposed on a first conductive substrate. The first biomolecule has a first nanoparticle disposed thereon. The system also includes a polymer disposed on the first conductive substrate. The polymer is catalytically generated on the first conductive substrate from a plurality of monomers, while the polymer is not substantially disposed on the first nanoparticle disposed on the first biomolecule. In addition, the system includes a detector capable of determining the presence of the first nanoparticle disposed on the first biomolecule.

An exemplary method, among others, can be broadly summarized by the following steps: providing a first target biomolecule disposed onto a first conductive substrate; providing a first nanoparticle; forming a first target complex that includes the first nanoparticle and the first target biomolecule disposed on the first conductive substrate; providing a solution of monomers; introducing the first target complex to a solution of monomers; catalyzing the polymerization of the monomers on the first target complex by applying a voltage to the first conductive substrat; forming a first polymerized target complex that includes a polymer disposed on the first target complex; and detecting the first polymerized target complex, which corresponds to the first target biomolecule.

Another exemplary method can be broadly summarized by the following steps: providing a first target biomolecule disposed onto a first conductive substrate; providing a first nanoparticle having a work function lower than the work function the first conductive substrate; forming a first target complex that includes the first nanoparticle and the first target biomolecule disposed on the first conductive substrate; providing a solution of monomers; introducing the first target complex to a solution of monomers; catalyzing the polymerization of the monomers on the first conductive substrate by applying a voltage to the first conductive substrate; forming a polymer on an area of the first conductive substrate where the first target complex is not located; and detecting the first target complex, which corresponds to the first target biomolecule.

Aspects of a number of preferred embodiments of the invention can be better understood with reference to the following drawings. The components in the drawings are not necessarily to scale, emphasis instead being placed upon clearly illustrating a number of preferred embodiments of the present invention. Moreover in the drawings, like reference numerals designate corresponding parts throughout the several views.
FIG. 1 is a flow diagram illustrating a representative embodiment of a process for determining the presence of a biomolecule.
FIG. 2 is a flow diagram illustrating a representative flow of an aspect of the process shown in FIG. 1.
FIG. 3 is a flow diagram illustrating a representative embodiment of a process for determining the presence of a biomolecule using imaging techniques.
FIGS. 4A through 4E are schematic diagrams of a process for determining the presence of a biomolecule using imaging techniques.
FIG. 5 is representative schematic diagram that depicts a polymer forming on a conductive substrate of FIGS 4A through 4E.
FIG. 6 is a flow diagram illustrating another representative embodiment of the process for determining the presence of a biomolecule using imaging techniques.
FIGS. 7A through 7D are representative schematic diagrams of another process for determining the presence of a biomolecule using imaging techniques.
FIG. 8 is a flow diagram illustrating a representative embodiment of a process for determining the presence of a biomolecule using electronic techniques.
FIGS. 9A through 9D are representative schematic diagrams of another process for determining the presence of a biomolecule using electronic measuring techniques.
FIG. 10 is a flow diagram illustrating a representative embodiment of a process for determining the presence of a biomolecule using mass measuring techniques.
FIGS. 11A through 11D illustrate a representative schematic diagram of another process for determining the presence of a biomolecule using mass measuring techniques.
FIGS. 12A and 12B illustrate a process for electrochemically amplifying a target biomolecule image for a biosensor system.

### DETAILED DESCRIPTION

As will be described in detail here, biosensor systems capable of determining the presence of biomolecules such as deoxyribouncleic acid (DNA), ribonucleic acid (RNA), nucleotides, oligonucleotides, nucleosides, proteins, peptides, polypeptides, and antibodies, for example are provided. In particular, several exemplary embodiments will be described that include determining the presence of biomolecules.

In general, determining the presence of a target biomolecule (i.e., the biomolecule of interest) is accomplished by detecting the presence of a polymer that can be associated with the target biomolecule. In this regard, a target nanoparticle is selectively disposed on the target biomolecule through a selective interaction (e.g., biological and/or chemical) between the target biomolecule and the target nanoparticle. The target biomolecule is "labeled," or "tagged," with the target nanoparticle by selectively binding the target biomolecule with the target nanoparticle via biological and/or chemical interactions. The biological interaction can include, for example, the bonding or hybridization between two biological functional groups. In this regard, the target nanoparticle can contain one or more biological functional groups that selectively interact with one or more biological functional groups of the target biomolecule. The chemical interaction can include, for example, reactions of one or more specific functional groups of the target biomolecule and the target nanoparticle.

Subsequently, the polymer is formed electrochemically from a solution of monomers on the target nanoparticle. The target nanoparticle is capable of catalyzing the polymerization of the monomer into the polymer. Then the polymer formed on the target nanoparticle can be detected using optical (imaging) techniques, electronic measurement techniques, and/or mass measurement techniques. The presence of the polymer indicates the presence of the target biomolecule.

As indicated above, the polymer is formed electrochemically on the target nanoparticle of the target complex. The polymer can include one or more polymers, where the one or more polymers are the same or different polymers. The electrochemical reaction can be facilitated by having the target biomolecule disposed upon a conductive substrate. The target biomolecule can be disposed onto the conductive substrate before or after the formation of the target complex. The target biomolecule attaches to the conductive substrate through biological and/or chemical interactions with a biological probe disposed on the conductive substrate, which is discussed in more detail below.

The electrochemical formation of the polymer on the target nanoparticle of the target complex can be accomplished by introducing a solution of monomers into the target complex. Subsequently, a voltage is applied to the conductive substrate, which catalyzes the formation of the polymer on the target complex. The target nanoparticle is used as a nucleation site to form the polymer catalytically on the target complex. This effect is driven by the difference in work functions between the target nanoparticle and conductive substrate. Work function can be defined as the minimum amount of energy required to remove an electron from the surface of a conductor.

For example, an anodic polymerization of pyrrole monomer is carried out on an indium tin-oxide (ITO) substrate, which has a work function of about 4.7eV, having gold target nanoparticles disposed thereon, which have a work function of about 5.1 eV. The polymer is formed on sites where the gold target nanoparticles are disposed because it has a higher work function than the ITO.

Thereafter, the polymer formed on the target complex can be detected using optical (imaging) techniques, electronic measurement techniques, and/or mass measurement techniques. Since the interaction between the target nanoparticle and the target biomolecule is selectively controlled, a direct correlation can be made between the detected polymer and the target biomolecule. Therefore, the target biomolecule can be qualitatively identified and/or quantitatively measured by detection of the polymer formed on the target complex.

In an alternative embodiment, the target nanoparticle can have a work function lower than the work function of the conductive substrate. In this embodiment, the polymer is formed on the conductive substrate rather than on the target nanoparticle. For example, an anodic polymerization of pyrrole is carried out on an indium tin-oxide (ITO) substrate, which have a work function of about 4.7eV, having silver target nanoparticles disposed thereon, which as a work function of about 4.2 eV. The polymer is formed on conductive substrate where the silver target nanoparticles are not disposed because the conductive substrate has a higher work function than the ITO.

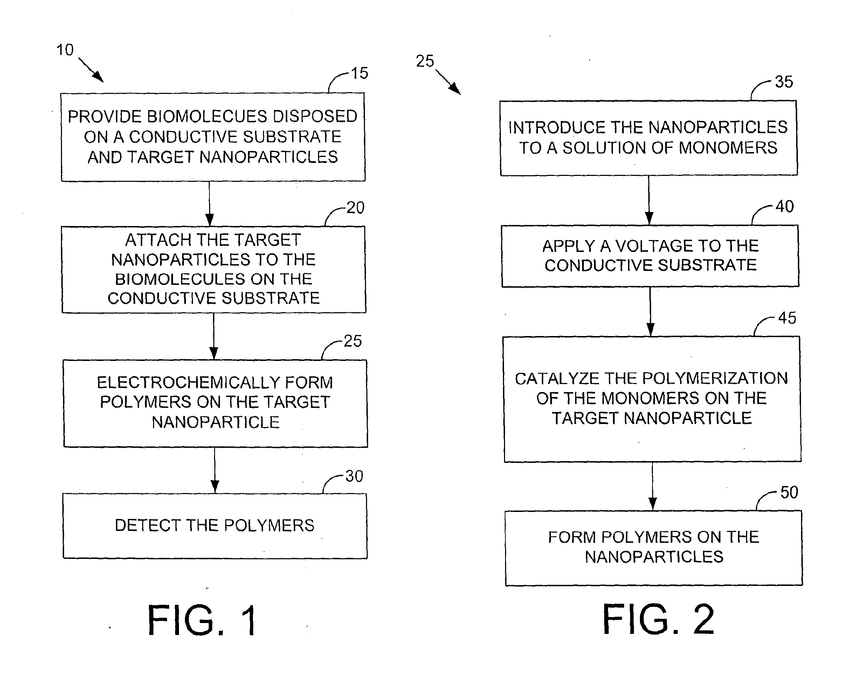

FIG. 1 is a flow diagram illustrating an exemplary process 10 for determining the presence of a target biomolecule. In block 15, a conductive substrate having target biomolecules disposed thereon and target nanoparticles are provided. Additional details regarding the conductive substrate, the target biomolecule, and the target nanoparticle will be discussed below. In block 20, the target nanoparticles are attached to the target biomolecules through biological and/or chemical interactions to form target complexes. In block 25, the polymer is electrochemically formed on the target complexes. In block 30, after forming the polymer on the target complexes, the polymer can be detected using imaging techniques, electronic measurement techniques, and/or mass measurement techniques, which are described in more detail below.

FIG. 2 is a flow diagram illustrating an exemplary aspect of the flow diagram shown in FIG. 1. In particular, FIG. 2 is a flow diagram illustrating block 25 of FIG. *1, i.e*., electrochemically forming the polymer on the target nanoparticles, in greater detail. In this regard, a solution including monomers is brought into contact with the target complexes attached to the conductive substrate, as shown in block 35. Additional details regarding the solution of monomers are discussed below. In block 40, a voltage is applied to the conductive support. In block 45, the voltage catalyzes the polymerization of the monomers on the target complexes. As a result, the polymer is formed on the target complexes, as shown in block 50.

In regard to the detection of the polymer by imaging techniques, the monomers can be selected so that the polymer has a high optical density. Typically, the polymer has high optical density (dark color) due to its molecular structure. When appropriate voltage is applied to the conductive substrate, most of the anodically-formed polymer is disposed at sites with higher work function. Thus, if the target nanoparticle has a higher work function than the conductive substrate, then the polymer should substantially deposit on the target nanoparicle, thereby forming a dark color at that position. For example, in a biosensor, the detected image of the polymer disposed on the target complex corresponds to the biomolecule image of the target biomolecule. Therefore, the target biomolecule can be qualitatively identified and/or quantitatively measured by imaging techniques.

In regard to the detection of the polymer by electrical measuring techniques, monomers are selected so that the polymer formed from the monomers is a conductive polymer. The formation of the conductive polymer results in a quantitative removal of electrons from monomers, which can be quantitatively monitored electronically. Thus, electronic measurement of the polymer corresponds to the presence of the target biomolecule.

In another embodiment of an electrical measuring technique, two conductive substrates having target complexes disposed thereon and located in close proximity to one another are introduced to a solution of monomers. As discussed above, the monomers form on the conductive polymer on the target nanoparticles of each of the target complexes once a voltage is applied to the conductive substrates. Due to the close proximity of the two conductive substrates, the polymer formed on each target complex grows and contacts a part of the second conductive substrate. As a result one or more electrical properties between the two conductive substrates can be tested.

For example, the resistance between the two conductive substrates can be tested. Prior to the formation of the conductive polymer, the resistance between the two conductive substrates is high since the two conductive substrates are separated from one another. Once the conductive polymer is formed on and around the nanoparticle complex on the first conductive substrate, it grows and contacts the second conductive substrate, resulting in a reduction in the resistance between the two conductive substrates. Thus, the resistance can be used as a test parameter for the measurement for the conductive polymer formed on the target complex. Therefore, the target biomolecule can be qualitatively identified and/or quantitatively measured using electronic measurement techniques.

In regard to the detection of the polymer by mass measurement techniques, the mass of the polymer can be used to determine the presence of the target biomolecule. In this regard, a conductive substrate having target complexes disposed thereon can be placed upon a mass measuring device, such as a quartz crystal microbalance, to determine the mass of the conductive substrate prior to forming the polymer thereon. Subsequently, the conductive substrate is introduced to a solution of monomers and then an appropriate voltage is applied to the conductive substrate. As discussed above, the monomers form the polymer on the target complexes. Thereafter, the mass of the conductive substrate having the polymer formed on the target complex can be measured using the mass measuring device. Thus, the increase in the mass can be used as a measurement for the formation of the polymer on the target complex. Therefore, the target biomolecule can be qualitatively identified and/or quantitatively measured using mass measurement techniques. In contrast, if the conductive substrate did not have target complexes disposed thereon, the polymer would not have formed and consequently there would not be a change in mass.

Having described biosensors and systems and methods for determining the presence of target biomolecules in general, four exemplary embodiments will know be described.

### Example 1

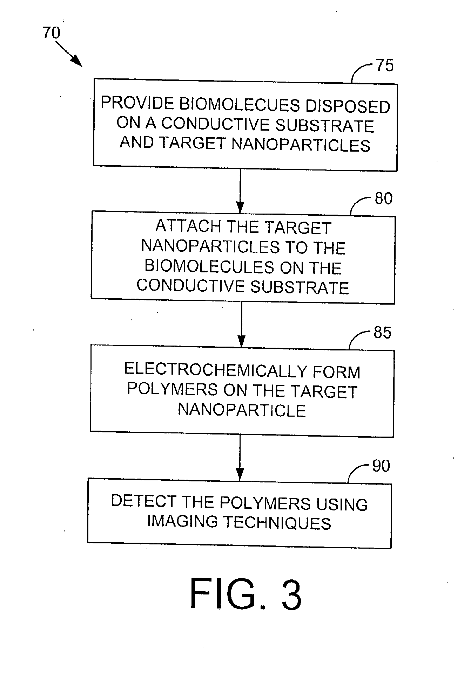

FIG. 3 is a flow diagram illustrating an exemplary process 70 for electrochemically amplifying a target biomolecule image for a biosensor system. In block 75, target biomolecules and target nanoparticles are provided. The target biomolecules are attached to a conductive substrate directly or indirectly via biological and/or chemical interactions. In addition, the target biomolecules can be attached to the conductive substrate in a known pattern. Additional details regarding the target biomolecules, the target nanoparticles, the conductive substrate, and how the target biomolecules are attached to the conductive substrate will be discussed below. In block 80, the target nanoparticles are disposed on the target biomolecules through a biological and/or a chemical interaction forming target complexes. In block 85, the target biomolecule image is amplified by selectively and electrochemically forming the polymer on the target complexes. In block 90, after forming the polymer on the target complexes, the polymer can be detected using imaging techniques.

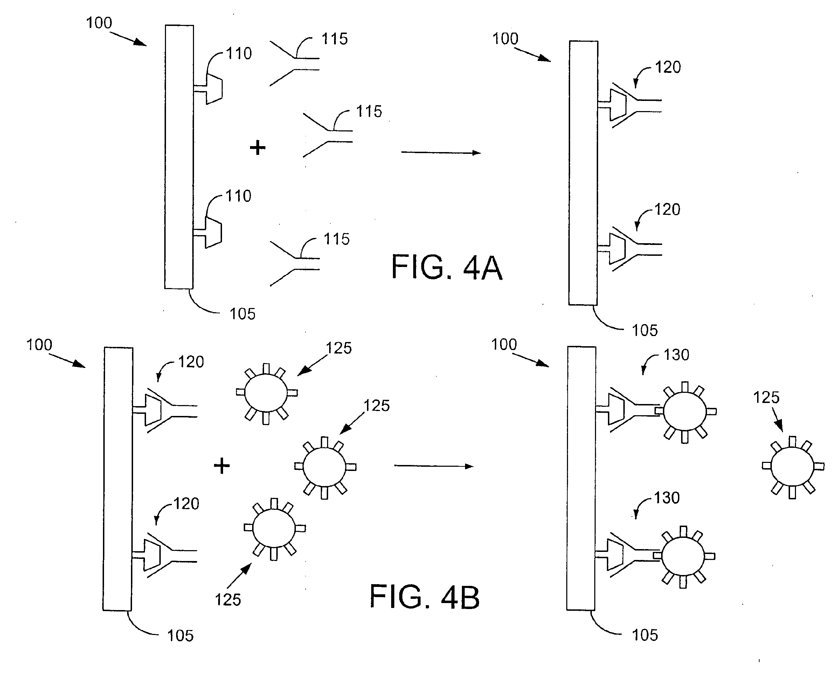

FIGS. 4A through 4E illustrate exemplary schematic diagrams of a process for electrochemically amplifying a target biomolecule image for a biosensor system 100. FIG. 4A illustrates the biosensor system 100, where probe biomolecules 110 are disposed on a conductive substrate 105. In addition, FIG. 4A illustrates introducing the target biomolecules 115 to the probe biomolecules 110 to form biomolecule complexes 120.

The probe biomolecules 110 are biomolecules that are capable of attaching to the conductive substrate. In addition, the probe biomolecules 110 are capable of selectively attaching to specific target biomolecules 115. For example, probe biomolecules include, but are not limited to, deoxyribouncleic acid (DNA), ribonucleic acid (RNA), nucleotides, oligonucleotides, nucleosides, proteins, peptides, polypeptides, and antibodies.

In this embodiment, the probe biomolecules 110 are attached to the conductive substrate 105 prior to being introduced to the target biomolecule 115. In other embodiments, the probe biomolecules 110 can be attached to the target biomolecule 115 prior to being attached to the conductive substrate 105.

The conductive substrate 105 can be any conductive substrate that has an affinity for the probe biomolecules. For example, the conductive substrate 105 may be formed of, for example, indium tin oxide (ITO) or gold. In addition, the conductive substrate 105 can be designed to have an affinity for different types of probe biomolecules so that multiple target biomolecules can be tested at one time. Furthermore, the surface of the conductive substrate 105 can be designed to interact with specific probe biomolecules at predetermined positions on the conductive substrate 105 to form known patterns. In this regard, patterning the conductive substrate with multiple probe biomolecules facilitates the testing of multiple target biomolecules at the same time. Thus, biosensors can be designed to test for one or more target biomolecules.

In other embodiments, the conductive substrate 105 can be designed to interact directly with the target biomolecule 115, which would preclude the need for probe biomolecules 110. Furthermore, the surface of the conductive substrate 105 can be designed to interact with specific biomolecules at predetermined positions on the conductive substrate. Thus, biosensors can be designed to test for one or more target biomolecules.

The target biomolecules 115 can include biomolecules such as, for example, deoxyribouncleic acid (DNA), ribonucleic acid (RNA), nucleotides, oligonucleotides, nucleosides, proteins, peptides, polypeptides, and antibodies.

FIG. 4B is a schematic diagram illustrating the introduction of target nanoparticles 125 to the biomolecule complexes 120 to form target complexes 130. As discussed above, the target nanoparticles 125 can be disposed on the target biomolecules 115 by selectively interacting with the target biomolecules 115. The interaction can be biological and/or a chemical interactions.

The target nanoparticles 125 can have a moiety and/or one or more functional groups disposed on the target nanoparticles 125 that have an affinity for a particular biomolecule type(s). Therefore, biosensors can be designed to determine the presence of one or more target biomolecules by exposing the biomolecules to one or more target nanoparticles having known affinities for certain target biomolecules.

In particular, the target nanoparticles 125 can be conjugated polymer nanoparticles such as, for example, 3,4-dioxyethylenethiophene (EDOT)/3-(N-pyrroyl) propionic acid in the size range of about 1 nanometer to about 1000 nanometers. Another type of target nanoparticle 125 includes nanoparticles made of materials such as, but not limited to, semiconductive materials (e.g., silicon, silica, and the like), and metals such as gold, silver, copper, nickel, or combinations thereof. In addition, the target nanoparticles made of semiconductive materials and metals may have one or more biological and/or chemical groups disposed thereon that have an affinity for one or more target biomolecules.

In general, the conductive substrate and the target nanoparticle are to be selected so that they have different work functions. As discussed above, the work function is defined as the minimum amount of energy required to remove an electron from the surface of a conductor. For example, when the conductive substrate is indiun-tin-oxide (ITO, which has a work function of about 4.7eV) and the target nanoparticle is gold (which has a work function of about 5.1eV), the 0.4 eV difference is sufficient to form polymer on the gold nanoparticles rather than the ITO conductive substrate. However, it should be noted that some polymer is formed on the ITO conductive substrate, but a greater percentage of polymer is formed on the gold nanoparticles so that a clear distinction can be detected.

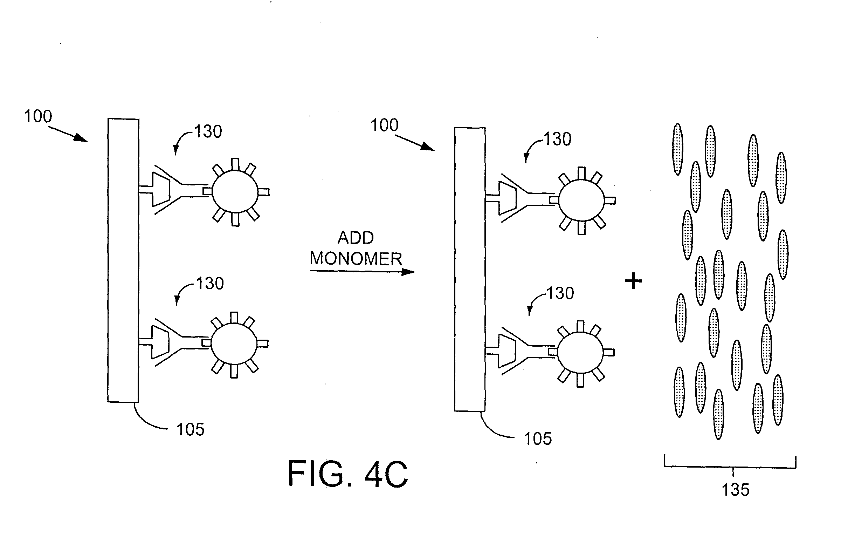

FIG. 4C is a schematic diagram illustrating the introduction a solution containing monomers 135 to the target complexes 130. The monomers 135 can be any monomer that can be formed into a polymer on the target nanoparticle 125 disposed on the target biomolecule. The monomers 125 can include, but are not limited to, monomers having one or more of the following functional groups: pyrrole, thiophene, aniline, fluorene, carbazole, catechol. In addition, the monomers 125 in solution can include one or more monomers 135 such as, for example, pyrrol and its derivatives, thiophene and its derivatives, aniline and its derivatives, fluorene and its derivatives, carbazole and its derivatives, and catechol (1,2-benzenediol) and its derivatives.

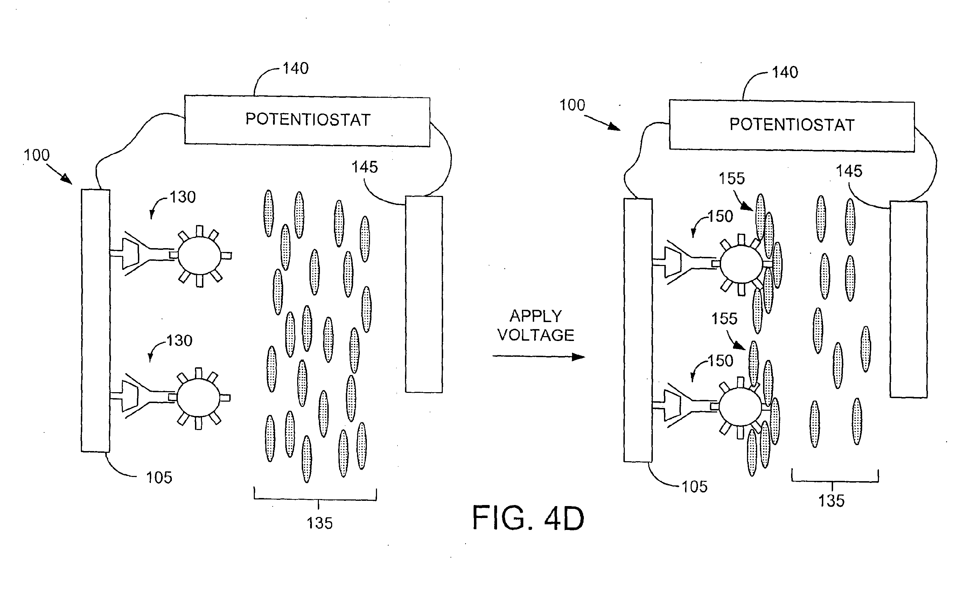

FIG. 4D is a schematic diagram illustrating application of a voltage to the conductive substrate 105 in the presence of the solution of monomers 135. The voltage (anodic) can be applied by a potentiostat 140 having a counter electrode 145 and a reference electrode (not shown) (e.g., silver/silver chloride (Ag/AgCl)). The applied voltage initiates the polymerization of the monomers 135 into the polymer 155 on the target complexes 130, thereby forming polymerized target complexes 150 on the conductive substrate 105. (General reference, Bard, *et al., Electrochemical Methods: Fundamentals and Application,* 2^{nd} Ed. 2000.)

For example, the formation of the polymer can be observed when the conductive substrate is an ITO conductive substrate and the target nanoparticle is a gold nanoparticle. When the anodic voltage is applied to the conductive substrate that has target complexes attached, electrons are moved from the target complexes to the conductive substrate due to the junction of the materials having different work function values. As a result, a positive charge made by the anodic voltage concentrates on the surface of the target nanoparticle of the target complex and the concentrated positive charge catalyzes the polymerization of monomers. Therefore, the monomers around the target complexes are oxidized first since the anodic voltage is not high enough to oxidize the conductive substrate. In this regard, the target nanoparticles act as nucleation sites for the polymerization of the monomers and, as a result, the polymer 155 selectively forms at the location where the target complexes 130 are disposed.

The polymer 155 formed on the polymerized target complex 150 can be any polymer 155 formed by the polymerization of the monomers 135 or combination of monomers. In particular, the polymer 155 can include, but is not limited to, polypyrroyl and derivatives thereof, polythiophene and derivatives thereof, polyaniline and derivatives thereof, polyfluorene and derivatives thereof, polycarbazole and derivatives thereof, polycatechol (1,2-benzenediol) and derivatives thereof, and combinations thereof

Using a common conjugated polymer can preserve the original distribution (image) of target complexes 130 on the surface of the conductive substrate 105. In addition, the common conjugated polymer has a high optical density, which can be used to enhance (amplify) the intensity of the original image of the target biomolecule 115 by forming the polymer 155 on the target complexes 130.

The potentiostat 140 can control the polymerization by adjusting the applied voltage, the scanning speed of the cyclic voltammogram, and the number of scanning cycles. In general, the applied voltage can range between about minus 1500 and 1500 millivolts (versus Ag/AgCl reference electrode). The voltammogram scan rate can vary from about 10 millivolts per second to about 300 millivolts per second. It should be noted, however, that the applied voltage, the scan rate, and the number of voltammogram scan cycles could be varied for each combination of materials, so that the background polymer deposition upon the conductive substrate 105 does not hamper the detection of the polymer formed on the target complexes 130.

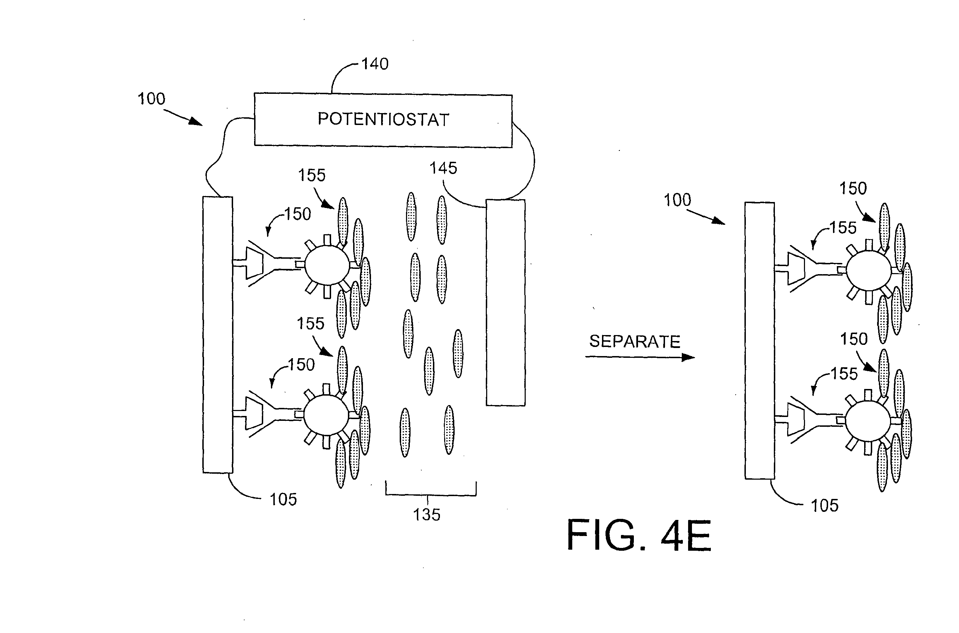

FIG. 4E is a schematic diagram illustrating the separation (*e.g.*, rinsing) of the monomers 135 and the remaining solution from the polymerized target complexes 150 disposed on the conductive substrate 105. Additional preparation and/or treatment of the polymerized target complexes 150 can be performed to facilitate their detection.

After sufficient time, the conductive substrate 105 is removed from the vessel and analyzed to determine the degree of polymerization. The biomolecule image of the conductive substrate 105 having the polymer 155 disposed on the target complexes 125 can be obtained using an imaging device such as, but not limited to, a standard flatbed scanner or any digital camera including Leaf EasyScan^{TM}, either of which can be controlled using a personal computer. Subsequently, the scanned biomolecule image can be analyzed using ImagePro^{TM} software, which can determine the densitometry of the scanned polymer image data. The densitometry data is a profile (*i.e.*, pixel points) of the area of the polymer image (the spot) as a function of optical density. In other words, each pixel point (*i.e.*, picture elements that define a unit area of the image) has a corresponding optical density. Therefore, a determination can be made whether the polymer 155 was formed on the each target complex 130 or not by measuring the optical density of the polymer image at each site where a probe biomolecule 110 was patterned. Thus, the target biomolecule 115 that corresponds to one or more of the probe biomolecules 110 is determined to be present if the image reveals that the polymer 155 was formed on each site with a corresponding probe biomolecule 110.

Additional preparation and/or treatment processes of the polymerized target complexes 150 can be performed to facilitate the detection of the polymerized target complexes 150. For example, the polymerized target complexes 150 can be exposed to chemicals and/or irradiation that enhance the ability of the polymer 155 to be detected.

Furthermore, the imaging technique can include radiochemical imaging techniques to compliment the imaging techniques discussed above. In some instances, the polymer 155 can be viewed with the human eye, thereby facilitating an inexpensive qualitative detection technique. For example, when a biosensor is designed to only form the polymer on a specific type of target biomolecule, visual detection of the polymer confirms the presence of the target biomolecule. Such a detection technique may be useful during off-site experimentation.

The concentrations of the monomer 135 used and the settings of the potentiostat 140 depend, at least in part, upon the chemical formulation of the monomer 135, the concentration of the monomer 135, the pH of the solution, the concentration of acid and/or base to achieve the appropriate pH, the temperature of the solution, the type of target nanoparticle 125, the conductive substrate 105, and the concentration of the target biomolecule 125. Therefore, the conditions under which the electrochemical amplification of the target biomolecule image is performed can vary, depending upon the design of the biosensor 100.

In another embodiment, when the conductive substrate has a higher work function than the target nanoparticle of the target complex, the polymer preferentially forms on the conductive substrate. For example, if the conductive substrate is ITO, which has a work function of about 4.7eV, and the target nanoparticle is a silver nanoparticle, which has a work function of about 4.2eV, then the polymer preferentially forms on the conductive substrate. In this regard, when the anodic voltage is applied to the conductive substrate 105 that has target complexes 130 disposed thereon, the electron is moved from the conductive substrate 105 to the target complexes 130. This occurs because of the junction between materials having different work function values. As a result, a positive charge made by the anodic voltage is concentrated on the surface of the ITO conductive substrate, which catalyzes the polymerization of monomers 135 into the polymer. Therefore, the monomers on the conductive substrate 105 that were not modified with the target complexes 130 are oxidized first since the anodic voltage is not high enough to oxidize the target complexes 130. Thus, target nanoparticles having lower work functions than the conductive substrate can be utilized to make the target complexes 130 act as inhibited sites for the polymerization of the monomers 135.

### Example 2

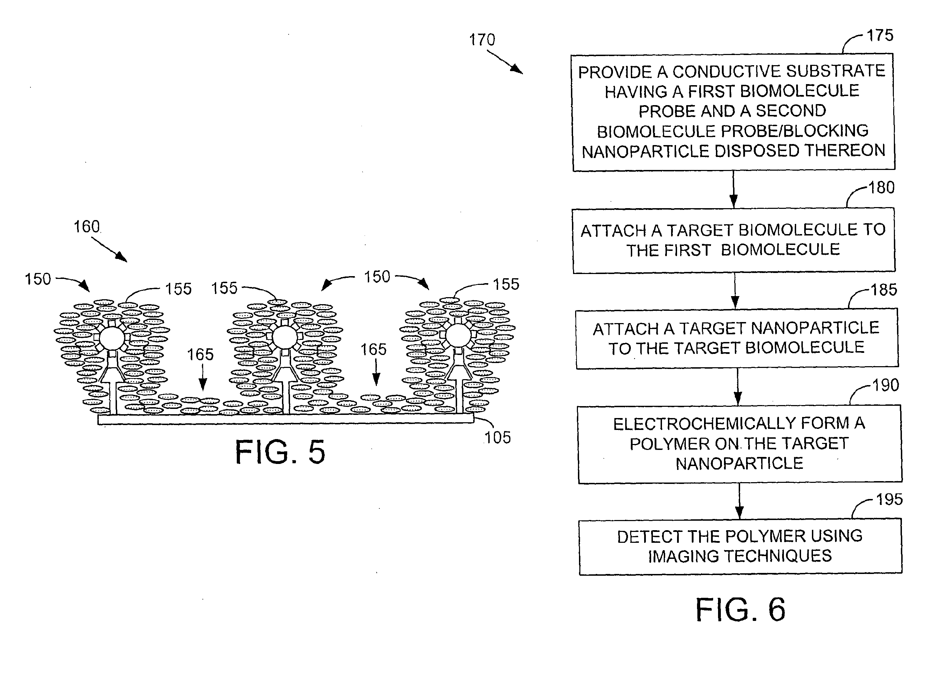

As discussed above, under certain electrochemical conditions the polymer may form on the conductive substrate, which can inhibit the detection of the target biomolecule because the polymer formed on the conductive substrate can "blur" or "hide" the biomolecule image of the target biomolecule. As FIG. 5 illustrates, under certain conditions the polymer 155 forms on the polymerized target complexes 150 and the polymer 165 forms on the conductive substrate 105.

The polymer can be inhibited from forming on the conductive substrate by disposing blocking nanoparticles on the surface of the conductive substrate. FIG. 6 is a flow diagram illustrating an exemplary process for forming a conductive substrate that inhibits the formation of the polymer on the conductive substrate. In block 175, a first probe biomolecule and a second probe biomolecule are disposed on a conductive substrate. In addition, the second probe biomolecule has a blocking nanoparticle disposed thereon. The first biomolecule, the second biomolecule, and the blocking nanoparticle are discussed in more detail below. In block 180, a target biomolecule is disposed on the first probe biomolecule. In block 185, a target nanoparticle is disposed onto the target biomolecule to form a target complex. In block 190, the conductive substrate is introduced to a solution of monomers. In addition, a polymer is electrochemically formed on the target complex forming a polymerized target complex. Furthermore, the polymer is inhibited from forming on the blocking nanoparticles. Subsequently, the polymer on the polymerized target complex is detected using imaging techniques, as shown in block 195.

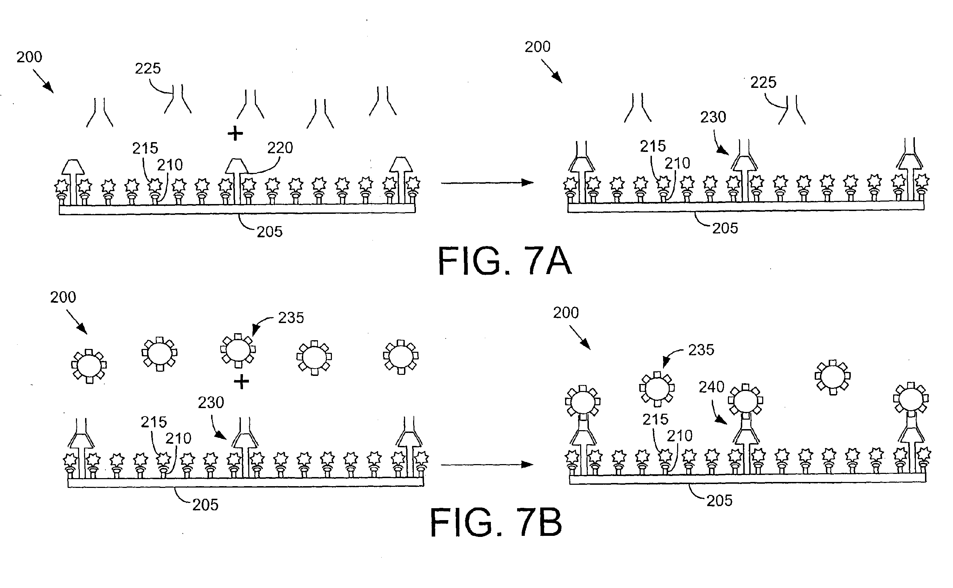

FIGS. 7A through 7D illustrate exemplary schematic diagrams of a process 200 for electrochemically amplifying a target biomolecule image for a biosensor system while inhibiting the formation of the polymer on the surface of the conductive substrates. FIG. 7A is a schematic diagram illustrating a conductive substrate 205 having a first probe biomolecule 220 disposed thereon. In addition, the conductive substrate 205 has a second probe biomolecule/blocking nanoparticle complex (210 and 215, respectively) disposed on the conductive substrate 205. The conductive substrate 205 is exposed to a solution of target biomolecules 225 having an affinity for the first probe biomolecule 220. The target biomolecules 225 attach via biological and/or chemical interactions to the first probe biomolecules 220, forming target biomolecule complexes 230.

The conductive substrate 205, the first probe biomolecule 220, the second biomolicule probe 210, and the target nanoparticle 225 are each similar to the conductive substrate 105, the probe biomolecule 110, and the target biomolecule 125, respectively, discussed in reference to FIGS. 4A through 4E.

The blocking nanoparticle 215 can be made of materials such as, but not limited to, semiconductive materials, metals such as silver, coated semiconductive materials, and coated metals. The coating can be biological and/or chemical to enhance the ability of the blocking nanoparticle 215 to interact with probe biomolecule 110 and/or the target biomolecule 125. As discussed above, the conductive substrate 205 and the blocking nanoparticle 215 are selected so that the work functions of each are different. In particular, the conductive substrate 205 and the blocking nanoparticle are selected so that the work function of the blocking nanoparticle 215 is lower than that of the conductive substrate 205. For example, when the conductive substrate 205 is ITO and the blocking nanoparticle is a silver nanoparticle, the 0.5eV difference is sufficient to inhibit the formation of polymer on the conductive substrate.

FIG. 7B is a schematic diagram illustrating introduction the conductive substrate 205 to a solution of target nanoparticles 235. As discussed above, the target nanoparticles 235 have an affinity for the target biomolecule 225 of the target biomolecule complex 230. The target nanoparticles 235 attach to the target biomolecules 225 to form target complexes 240. The target biomolecules 225 are similar to the target nanoparticles 125 discussed above in reference to FIGS. 4A through 4E.

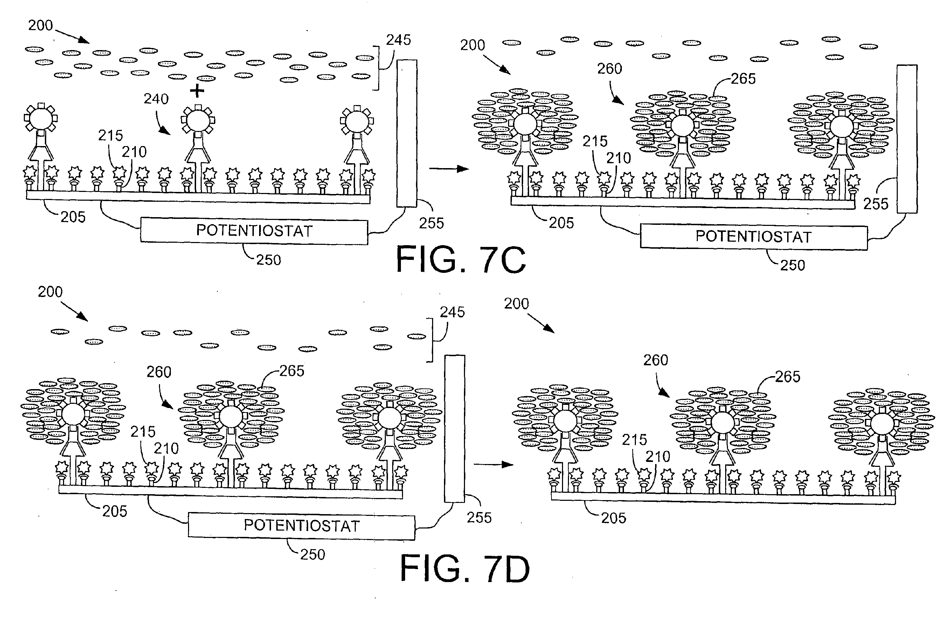

FIG. 7C is a schematic diagram illustrating application of a voltage to the conductive support 205 in the presence of the solution of monomers 245. The anodic voltage can be applied by a potentiostat 250 having a counter electrode 255 and a reference electrode (not shown) (e.g., silver/silver chloride (Ag/AgCl)). The applied voltage initiates the polymerization of the monomers 245 into the polymer 265 on the target complexes 240, in a manner similar to that discussed in reference to FIGS. 4A through 4E, thereby forming polymerized target complexes 260 on the conductive substrate 205. In addition, the polymer 265 does not substantially form on the conductive substrate 205 because of the presence of the blocking nanoparticles 215. The monomers 245 and the polymer 265 are similar to the monomers 135 and the polymer 155 discussed above in reference to FIGS. 4A through 4E.

FIG. 7D is a schematic diagram illustrating separation (e.g., rinsing) of the monomers 245 and the remaining solution from the target biomolecules 225 disposed on the conductive substrate 205 so that imaging techniques can be used to determine the presence of the target biomolecules 225. As discussed above, additional preparation and/or treatment of the polymerized target complexes 260 can be performed to facilitate the detection of the polymerized target complexes 260.

### Example 3

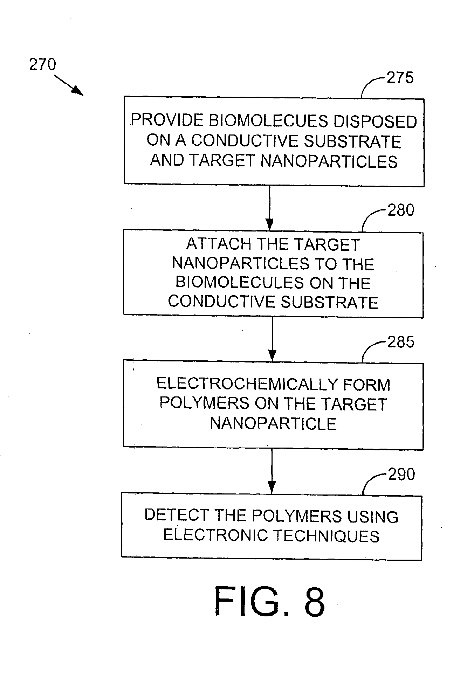

FIG. 8 is a flow diagram illustrating an exemplary process 270 for using electronic measuring techniques to determine the presence of a target biomolecule for a biosensor system. In block 275, the target biomolecules and the target nanoparticles are provided in a vessel. The target biomolecules are attached to a conductive support via biologicand and/or chemical interations. In block 280, the target nanoparticles are disposed on the target biomolecules through biological and/or chemical interactions. In block 285, the polymer is electrochemically formed on the target nanoparticles. In block 290, after forming the polymer on the target nanoparticles, the polymer can be detected using electronic measuring techniques.

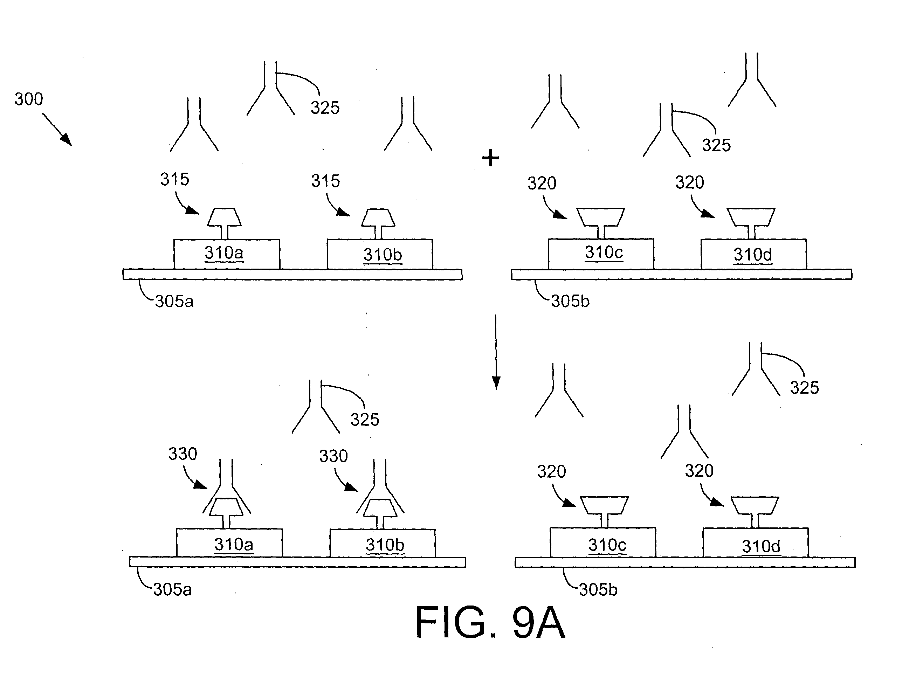

FIG. 9A is a schematic diagram illustrating the introduction of the target biomolecules 325 to a first structure 305a and a second structure 305b. The first structure 305a includes the first conductive substrate 310a and a second conductive substrate 310b. The second structure 305b includes the third conductive substrate 310c and a fourth conductive substrate 310d. The first conductive structure 310a and the second conductive structure 31 0b have first probe biomolecules 315. disposed thereon. In addition, the first conductive structure 310a and the second conductive structure 310b are located in close proximity to one another. The third conductive structure 310c and the fourth conductive structure 310d have second probe biomolecules 320 disposed thereon. The first probe biomolecules 315 and the target biomolecules 325 have an affinity for one another, while the second probe biomolecules 320 and the target biomolecules 325 do not have an affinity for one another. Therefore, the target biomolecules 325 attach to the first probe biomolecules 315 to form target biomolecule complexes 330.

The first structure 305a and the second structure 305b can be made of insulator materials such as, but not limited to, soda glass, quartz, and polymer materials. The first, second, third, and fourth conductive substrates 310a, 310b, 310c, and 310d; the first probe biomolecule 315 and the second probe biomolecule 320; and the target biomolecule 325 are similar to the conductive substrate 105, the probe biomolecule 110, and the target biomolecule 125, respectively, discussed in reference to FIGS. 4A through 4E.

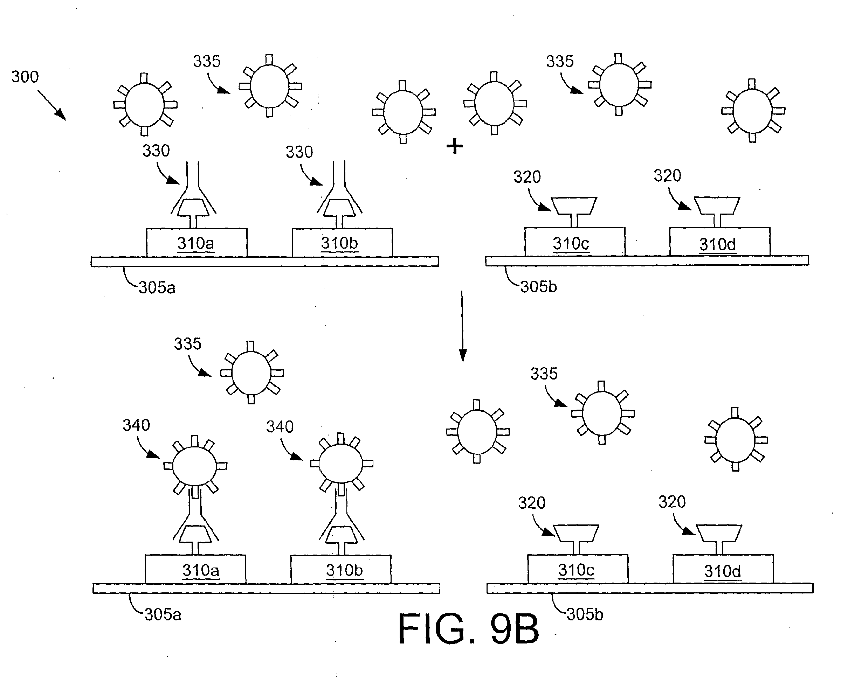

FIG. 9B is a schematic diagram illustrating the introduction of target nanoparticles 335 to the first, second, third, and fourth conductive substrates 310a, 310b, 310c, and 310d. The target nanoparticles 335 and the target biomolecule complexes 330 have an affinity for one another, while the target nanoparticles 335 and the second probe biomolecules 320 do not have an affinity for one another. Therefore, the target nanoparticles 335 attach to the target biomolecule complexes 330 to form target complexes 340. The target nanoparticles 335 are similar to the target nanoparticles 125 dicussed in reference to FIGS. 4A through 4E.

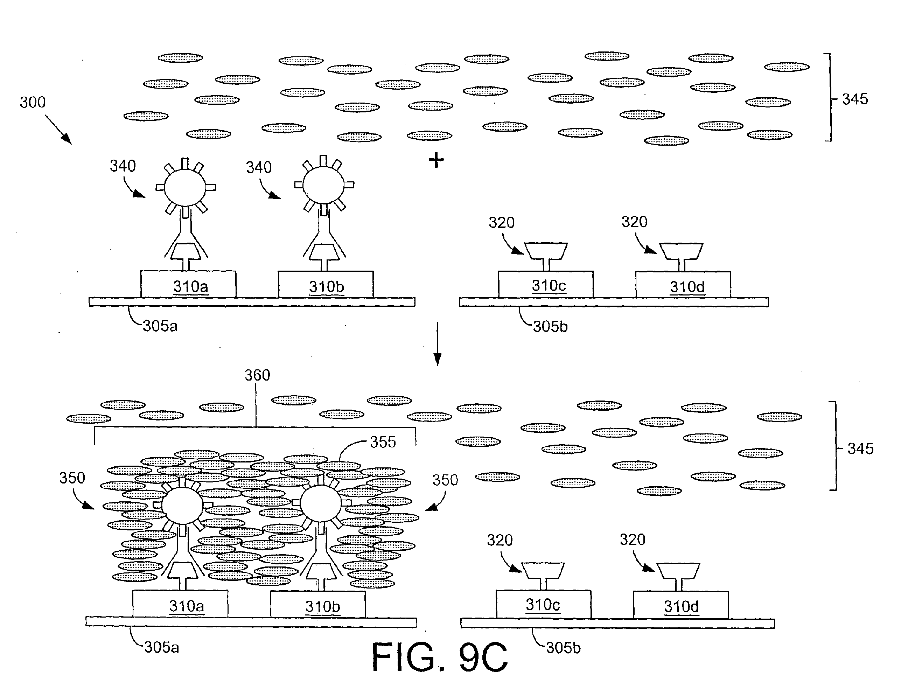

FIG. 9C is a schematic diagram illustrating the introduction of a solution of monomers 345 to the first, second, third, and fourth conductive substrates 310a, 310b, 310c, and 310d. In addition, a voltage is applied to first, second, third, and fourth conductive substrates 310a, 310b, 310c, and 310d using a potentiostat (not shown) in a manner similar to FIGS. 4A through 4E shown previously. The target nanoparticles 335 disposed on the target biomolecule complexes 340 act as nucleation sites for the electrochemical polymerization of the monomers 345. Thus, the conductive polymer 355 forms on the target nanoparticles 335 to form polymerized target nanoparticles 350. Since the first and second conductive substrates 310a and 310b are in close proximity to one another, the conductive polymer 355 from each polymerized target nanoparticle complex 350 merge into one mass of conductive polymer 360 that encompasses both polymerized target nanoparticle complexes 350. In contrast, the conductive polymer 355 are not substantially formed on the third and fourth substrates 310c and 310d since they do not include target nanoparticles 335. The monomers 345 and the polymer 355 are similar to the monomers 135 and the polymer 155 discussed in reference to FIGS. 4A through 4E.

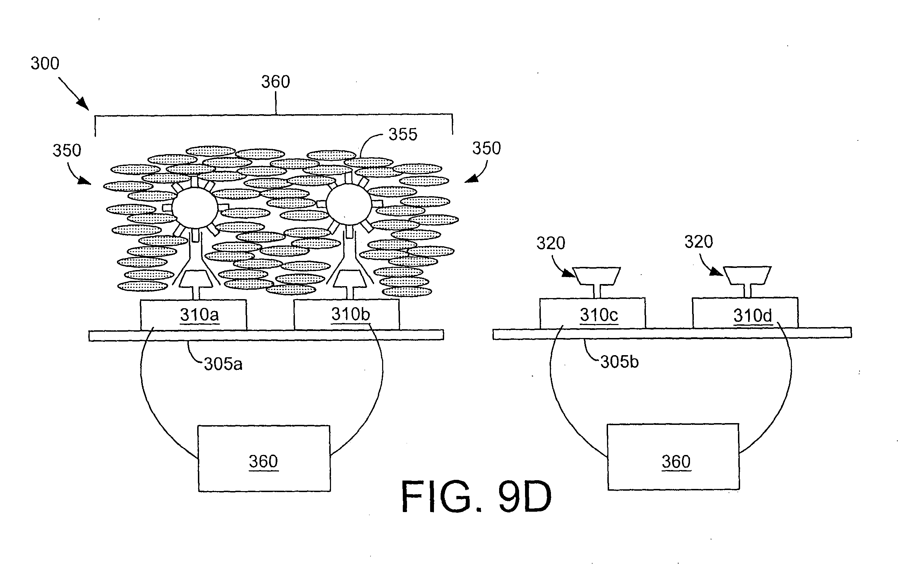

FIG. 9D is a schematic diagram illustrating attachment of contacts to the first and second conductive structures 310a and 310b, where the contacts are connected to a device 360 capable of measuring one or more electrical properties between the first and second conductive structures 310a and 310b to determine the presence of the polymer 355. For example, an ohmmeter can be attached to the first and second conductive structures 310a and 310b to measure the resistance between the first and second conductive structures 310a and 310b. Since the conductive polymer 355 of the polymerizied target complexes 350 disposed on the first and second conductive structure 310a and 310b have merged into one mass of conductive polymer 360, the resistance between the first and second conductive structures 310a and 310b is reduced. In contrast, the resistance between the third and fourth conductive structures 310c and 310d is high since there is no conductive connection between the these conductive structures. Thus, the presence of the conductive polymer 355 *(i.e.,* the target biomolecules 325) can be detected using electronic measuring techniques.

### Example 4

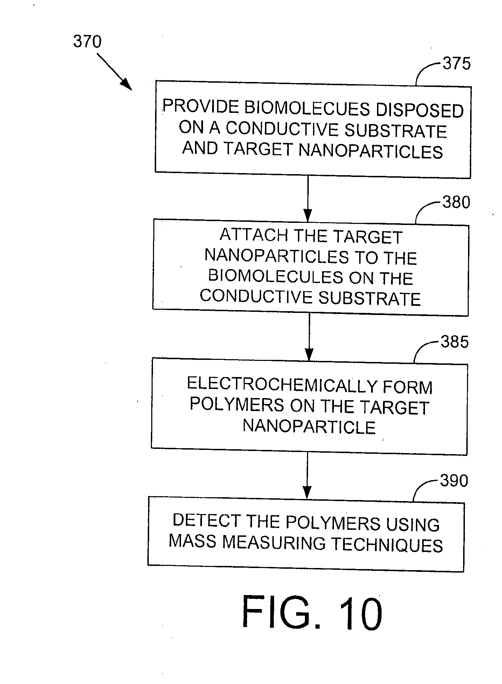

FIG. 10 is a flow diagram illustrating an exemplary process 370 for using mass measuring techniques to determine the presence of target biomolecules for a biosensor system. In block 375, the target biomolecules and the target nanoparticles are provided. The target biomolecules are attached to a conductive support directly or indirectly via biological and/or chemical interactions. In block 380, the target nanoparticles are disposed on the target biomolecules through a biological and/or a chemical interaction to form a target complex. In block 385, the polymer is electrochemically formed on the target complex. In block 390, after forming the polymer on the target complex, the polymer can be detected using mass measuring techniques.

FIG. 11A is a schematic diagram illustrating the application of the target biomolecules 415 with a first conductive substrate 410a and a second conductive substrate 410b. The first and second conductive substrates 410a and 410b are each positioned on a mass measuring device 405a and 405b such as a quartz crystal microbalance. The first conductive substrate 410a has first probe biomolecules 420 disposed thereon. The second conductive substrate 410b has second probe biomolecules 425 disposed thereon. The first probe biomolecules 420 and the target biomolecules 415 have an affinity for one another, while the second probe biomolecules 425 and the target biomolecules 415 do not have an affinity for one another. Therefore, only the target biomolecules 415 attach to the first probe biomolecules 420 to form target biomolecule complexes 430.

The first conductive substrate 410a and the second conductive substrate 410b, the first probe biomolecules 420 and the second probe biomolecules 425, and the target biomolecule 425 are similar to the conductive substrate 105, the probe biomolecules 110, and the target biomolecule 125, respectively, discussed in reference to FIGS. 4A through 4E.

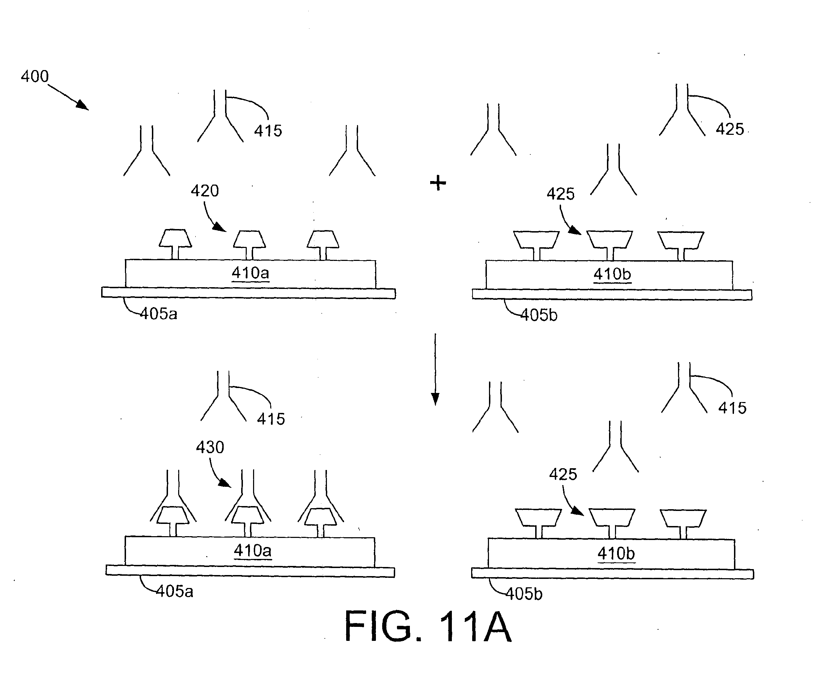

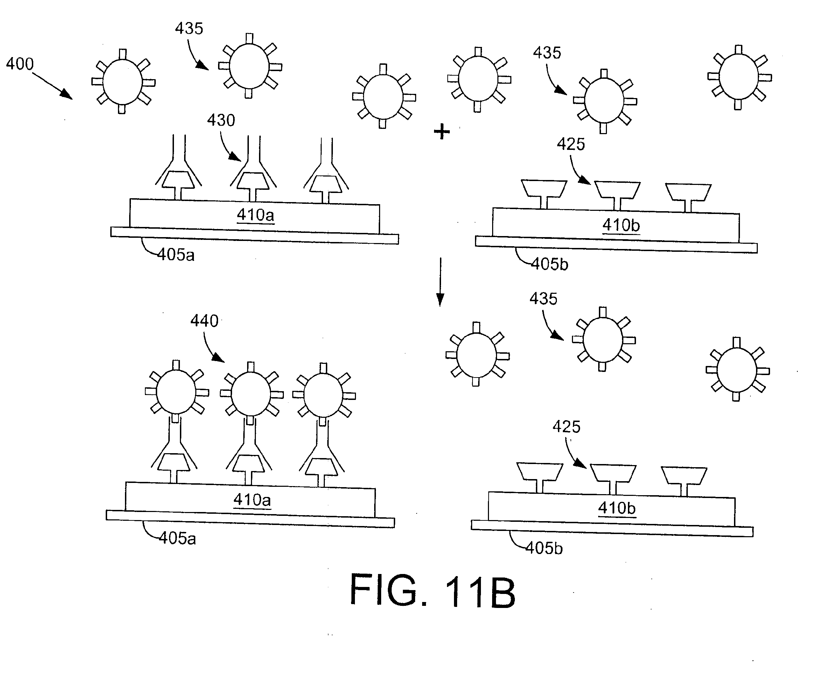

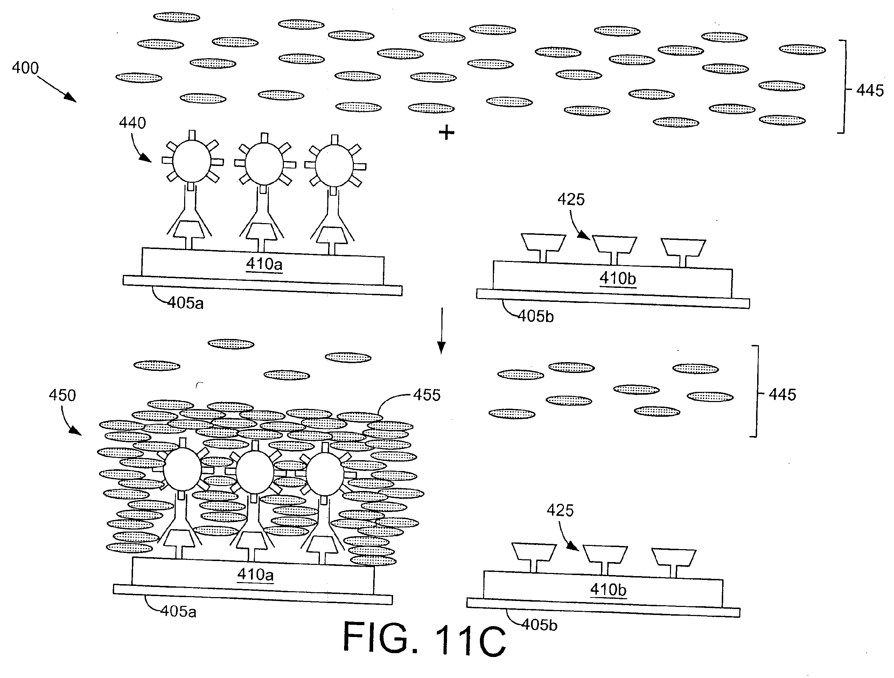

FIG. 11B is a schematic diagram illustrating the introduction of the target nanoparticles 435 to the first conductive substrate 410a and the second conductive substrate 410b. The target nanoparticles 435 and the target biomolecule complexes 430 have an affinity for one another, while the target nanoparticles 435 and the second probe biomolecules 425 do not have an affinity for one another. Therefore, the target nanoparticles 435 attach to the target biomolecule complexes 430 to form target complexes 440. The target nanoparticles 435 are similar to the target nanoparticles 125 discussed in reference to FIGS. 4A through 4E.

FIG. 11C is a schematic diagram illustrating the introduction of a solution of monomers 445 to the first conductive substrate 410a and the second conductive substrate 410b. In addition, a voltage is applied to the first conductive substrate 410a and the second conductive substrate 410b using a potentiostat (not shown) in a manner similar to FIGS. 4A through 4E shown previously. The target nanoparticles 435 disposed on the target biomolecule complexes 440 act as a nucleation site for the polymerization of the monomers 445 into the polymer 455. Thus, the polymer 455 forms on the target biomolecule complexes 440 to form polymerized target complexes 450. In contrast, the polymer 455 does not substantially form on the second probe biomolecules 425. The monomers 445 and the polymer 455 are similar to the monomers 135 and the polymer 155 discussed in reference to FIGS. 4A through 4E.

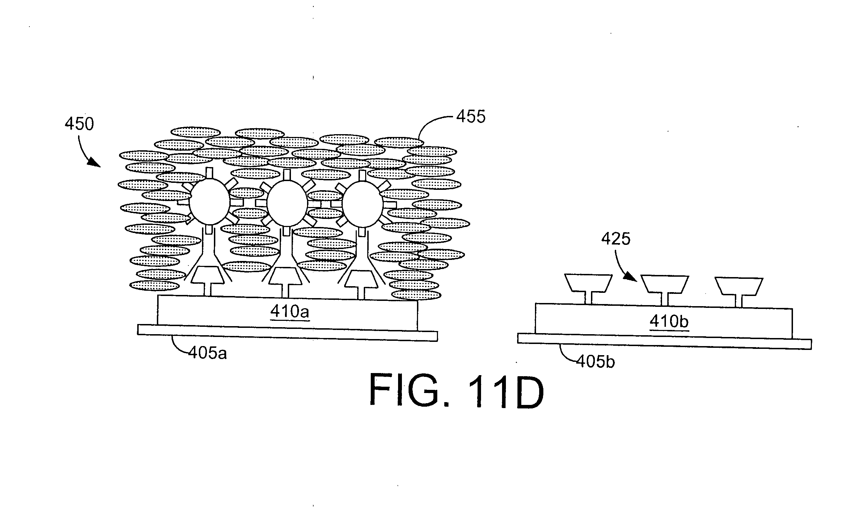

FIG. 11D is a schematic diagram illustrating measurement of the mass of the first conductive substrate 410a and the second conductive substrate 410b. The mass of the polymer 455 formed on the polymerized biomolecule complexes 450 can be measured with the mass measuring device 405a. Therefore, the presence of the polymer 455 (*i*.*e*., the target biomolecules 425) can be detected using the increased mass of the first conductive substrate 410a caused by the formation of the polymer 455 on the target nanoparticles 435 of the polymerized target complexes 450.

### Example 5

Initially, two ITO conductive substrates were treated with N-[3-(trimethoxysilyl)propyl]ethylenediamine (both of which can be purchased from Sigma-Aldrich (Milwaukee, WI)) solution in isopropanol to modify the surface of the conductive substrate to have an affinity for amino groups. Then the ITO conductive substrates were spotted with a solution of sulfo-N-hydroxysucciniimide(NHS)-long chain(LC)-LC-biotin, which can be purchased from Pierce Biotechnology, Inc. (Rockford, Illinois), to attach the biotin probe biomolecule to the ITO conductive substrate. Biotin is a functional group that specifically interacts with avidin or streptavidin, while the NHS group reacts with amino groups on ITO surface. The sulfo-NHS-LC-LC-biotin solution (about 100 mg/mL) was mixed with a solution with of a carbonate buffer (3.18g Na₂CO₃, 5.86g NaHCO₃, 0.4g NaN₃ in 200 mL H₂O with pH = 9.6). The resultant solution was spotted onto the ITO conductive substrates using a manual pipetor. Then the surface was incubated in a moist chamber at room temperature for about 2 hours.

Next, the ITO conductive substrates were rinsed with deionized water. The first ITO conductive substrate was then incubated in a solution of gold-ExtrAvidin conjugate target nanoparticles, which can be purchased from Sigma-Aldrich, for 30 minutes at about 4°C, and subsequently rinsed with water. The second ITO conductive substrate was not incubated with the gold-ExtrAvidin conjugate target nanoparticles, which was done as a control to evaluate the biosensor.

Then both ITO conductive substrates were introduced to an aqueous solution containing 0.5% pyrrole monomer and 0.5% poly(4-styrenesulfonate). A cyclic voltammogram was performed on both ITO conductive substrates. In this regard, a potential was applied with a CS-1200 potentiostat, which can be purchased from Cypress Systems (Lawrence, KS). The applied potential that was scanned between about 300 mV and 1300 mV (vs. Ag/AgCl) at 200 mV/s for 99 cycles and the change in anodic current over time was recorded. After the ITO conductive substrates were treated, each ITO conductive substrate was rinsed with deionized water, and dried with nitrogen spray.

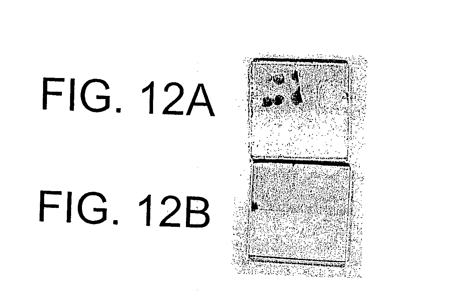

FIGS. 12A and 12B illustrate a process for electrochemically amplifying a target biomolecule image for a biosensor system. In particular, FIG. 12A shows the first ITO conductive substrate spotted biotin layer and having a polypyrrole polymer disposed on the gold-ExtrAvidin target nanoparticles. FIG. 12B shows the second ITO conductive substrate spotted biotin layer that does not have the gold-ExtrAvidin target nanoparticles disposed on the biotin layer, and consequently, the formation of the polypyrrole polymer does not occur.

In particular, FIG. 12A shows six dark and nearly circular areas, which corresponds to the biotin spots on the ITO conductive substrate having the polypyrrole disposed on the gold-ExtrAvidin target nanoparticles. In contrast, FIG. 12B does not show any dark areas on the ITO conductive substrate because the polypyrrole polymer was not formed. It should be noted that the dark spot located in the lower left hand comer of FIG. 12B is due to formation of the polymer on the edge of the ITO conductive substrate, which is not related to formation of polymer on the biotin layer of the ITO conductive substrate. Thus, the formation of polypyrrole polymer in FIG. 12A illustrates that the presence of a target biomolecule can be determined.

It should be noted that the six dark areas can be observed with the naked eye. Thus, this biosensor can be used to quickly test for the presence of a target biomolecule. Alternatively, an image of the six dark areas can be taken by a camera and/or the image can be scanned into a computer using scanner for additional processing, which was discussed in more detail above.

## Claims

1. A method of determining the presence of biomolecules, comprising:
providing a first target biomolecule 115, a first target nanoparticle 125, and a polymer 155;
forming a first polymerized target complex 150 electrochemically on a conductive substrate 105, wherein the first polymerized target complex 150 includes the first target biomolecule 115, the first target nanoparticle 125, and the polymer 155, wherein the polymer 155 is disposed on the first target nanoparticle 125, wherein the first target nanoparticle 125 is disposed on the first target biomolecule 115, and wherein the first target biomolecule 125 is disposed on the conductive substrate 105; and
detecting the first polymerized target complex 150, which corresponds to the first target biomolecule 115.

2. The method of claim 1, wherein detecting the polymerized target complex 150 comprises:
detecting the polymerized target complex 150 by imaging techniques capable of detecting the polymer 155 disposed on the polymerized target complex 150.

3. The method of claim 1, further comprising:
detecting the polymerized target complex 150 by determining the change in mass of the first conductive substrate 105 having the polymerized target complex 150 disposed thereon.

4. The method of claim 1, further comprising:
detecting the polymerized target complex 150 via an electronic measurement technique.

5. The method of any preceding claim, wherein the first nanoparticle 125 is selected from a gold nanoparticle, a copper nanoparticle, and a nickel nanoparticle.

6. The method of any preceding claim, wherein the work function of the first conductive substrate 105 is lower than the work function of the first target nanoparticle 125.

7. A biosensor system for determining the presence of biomolecules, comprising:
a first biomolecule 115 disposed on a first conductive substrate 105;
a first nanoparticle 125 disposed on the first biomolecule 115;
a polymer 155 disposed on the first nanoparticle 125, wherein the polymer 155 is catalytically generated on the first nanoparticle 125 from a plurality of monomers 135; and
a detector 140 capable of determining the presence of the first biomolecule 115.

8. The system of claim 7, wherein the first nanoparticle 125 includes a gold nanoparticle, a nickel nanoparticle, and a copper nanoparticle.

9. A biosensor system for determining the presence of biomolecules, comprising:
a first biomolecule 225 disposed on a first conductive substrate 205;
a first nanoparticle 235 disposed on the first biomolecule 225;
a polymer 265 disposed on the first conductive substrate 205, wherein the polymer 265 is catalytically generated on the first conductive substrate 205 from a plurality of monomers 245, and wherein the polymer 265 is not substantially disposed on the first nanoparticle 235 disposed on the first biomolecule 225; and
a detector 250 capable of determining the presence of the first nanoparticle 235 disposed on the first biomolecule 225.

10. The system of claim 9, wherein the first nanoparticle 235 includes a silver nanopaticle.
